# EUROPEAN PATENT SPECIFICATION

(11) **EP 3 066 122 B1**
(45) Date of publication and mention of the grant of the patent: **14.04.2021**
(21) Application number: 14860426.7
(22) Date of filing: 05.11.2014
(51) Int. Cl.: C07K 16/00, C12P 21/08, C07K 17/00, C07K 17/14, A61K 39/395, A61K 39/00, C07K 16/18, G01N 33/577

(54) **PRE-HAPTOGLOBIN-2 MONOCLONAL ANTIBODIES AND USES THEREOF**
MONOKLONALER PRÄ-HAPTOGLOBIN-2-ANTIKÖRPER UND VERWENDUNGEN DAVON
ANTICORPS MONOCLONAUX ANTI-PRÉ-HAPTOGLOBINE-2 ET LEURS UTILISATIONS

(30) Priority: 05.11.2013 US 201361900001 P
(43) Date of publication of application: 14.09.2016
(73) Proprietor: Bio-Rad Laboratories, Inc., Hercules, CA 94547 (US)
(72) Inventor: FLANAGAN, John, Hercules California 94547 (US); WALKER, Roger, Hercules, California 94547 (US); DU PATY, Emilie, 34184 Montpellier Cedex 4 (FR); GALEA, Pascale, J34830 Jacou (FR); RIEUNIER, Francois, 92430 Marnes-La Coquette (FR); LAUNE, Daniel, 34790 Grabels (FR)
(74) Representative: dompatent von Kreisler Selting Werner - Partnerschaft von Patent- und Rechtsanwälten mbB
(86) International application number: PCT/US2014/064055
(87) International publication number: WO 2015/069718

(56) References cited:
- WO-A2-2013/090259
- KR-B1- 100 737 326
- KR-B1- 100 737 326
- US-A1- 2011 183 923
- US-A1- 2011 183 923
- US-A1- 2012 107 329
- "Zonulin ELISA Kit", Immundiagnostik, 1 January 2010 (2010-01-01), pages 1-27, XP055218524, Retrieved from the Internet: URL:http://www.immundiagnostik.com/pdf/Zon ulin.pdf [retrieved on 2015-10-06]
- FLANAGAN J J ET AL: "Development of monoclonal antibodies to pre-haptoglobin 2 and their use in an enzyme-linked immunosorbent assay (ELISA)", JOURNAL OF IMMUNOLOGICAL METHODS, ELSEVIER SCIENCE PUBLISHERS B.V.,AMSTERDAM, NL, vol. 406, 25 February 2014 (2014-02-25), pages 34-42, XP029024220, ISSN: 0022-1759, DOI: 10.1016/J.JIM.2014.02.009
- 'Zonulin ELISA Kit', [Online] 2010, page 16, XP055218524 Retrieved from the Internet: <URL:http://www.immundiagnostik.com/pdf/Zon ulin.pdf> [retrieved on 2015-02-03]

## Description

### BACKGROUND OF THE INVENTION

Haptoglobins bind to free hemoglobin in plasma (see, e.g., Anderson et al., Nature 489(7416):456-9 (2012)). Haptoglobin is made up of two α- and two β-chains, connected by disulfide bridges. The chains originate from a common precursor protein ("pre-Haptoglobin"), which is proteolytically cleaved during protein synthesis.

The Haptoglobin gene (*Hp*) exists in two allelic forms in the human population, called *Hp1* and *Hp2,* the latter one having arisen due to the partial duplication of *Hp1* gene. Three genotypes of *Hp,* therefore, are found in humans: *Hp1-1, Hp2-1,* and *Hp2-2.* Different *Hp* genotypes have been shown to bind hemoglobin with different affinities, with *Hp2-2* being the weakest binder.

Accordingly, there are two possible Haptoglobin proteins that can be detected (Haptoglobin-1 and Haptoglobin-2) with precursors of each (pre-Haptoglobin-1 and pre-Haptoglobin-2) also generated (s*ee, e.g.,* Polticelli, F., et al., FEBS J. 275(22):5648-56 (2008) and WO 2013/090259).

Recently, it has been determined that pre-Haptoglobin-2 has some biological activity similar to a toxin made by *Vibrio cholera,* zonula occludens toxin (Zot) (*see,* US Patent Publication 2012/0107329). On the other hand the in vitro determination of Zonulin, a human protein similar to Zot in serum, EDTA-plasma and stool is possible with a Zonulin ELISA kit of Immundiagnostik AG (http://www.immundiagnostik.com/pdf/Zonulin.pdf). Furthermore, peptide antagonists of Zonulin and its use are disclosed in US 2011/0183923.

### BRIEF SUMMARY OF THE INVENTION

Monoclonal antibodies (e.g., isolated antibodies) that bind pre-Haptoglobin-2 are provided.

In particular, the antibodies bind human pre-Haptoglobin-2 but do not bind human pre-Haptoglobin-1, human Haptoglobin-1, or human Haptoglobin-2 as determined by surface plasmon reasonance, wherein the monoclonal antibody specifically binds GYVEHSVRY (SEQ ID NO:1). The monoclonal antibody may bind KPPEIAHGYVEHSVRYQCKNYYK (SEQ ID NO:2). The monoclonal antibody may bind KPPEIAHGYVEHSVR (SEQ ID NO:3), PPEIAHGYVEHSVRY (SEQ ID NO:4), PEIAHGYVEHSVRYQ (SEQ ID NO:5), EIAHGYVEHSVRYQC (SEQ ID NO:6), IAHGYVEHSVRYQCK (SEQ ID NO:7), AHGYVEHSVRYQCKN (SEQ ID NO:8), HGYVEHSVRYQCKNY (SEQ ID NO:9), GYVEHSVRYQCKNYY (SEQ ID NO:10), and/or YVEHSVRYQCKNYYK (SEQ ID NO:11). The monoclonal antibody may bind one or more peptide listed above but may not bind PKPPEIAHGYVEHSV (SEQ ID NO:12) or VEHSVRYQCKNYYKL (SEQ ID NO:13).

In some embodiments, the monoclonal antibody binds pre-Haptoglobin-2 with a KD of less than 25 or 10 nM. In some embodiments, the monoclonal antibody is linked to a detectable label. In some embodiments, the detectable label is biotin. In some embodiments, the monoclonal antibody is linked to a solid support. The monoclonal antibody may be a chimeric antibody.

In some embodiments, the monoclonal antibodies bind non-reduced human pre-Haptoglobin-2 but do not significantly bind reduced human pre-Haptoglobin-2. In some embodiments, the monoclonal antibody binds pre-Haptoglobin-2 with a KD of less than 25 or 10 nM. In some embodiments, the monoclonal antibody is linked to a detectable label. In some embodiments, the detectable label is biotin. In some embodiments, the monoclonal antibody is linked to a solid support.

Also provided are kits comprising one or more monoclonal antibody or antibody pair as described herein. The kit comprises a monoclonal antibody as described above, wherein the monoclonal antibody is linked to a solid support. In some embodiments, the kit further comprises a second antibody that binds human pre-Haptoglobin-2, wherein the monoclonal antibody linked to the solid support and the second antibody can simultaneously bind to human pre-Haptoglobin-2. In some embodiments, the second monoclonal antibody is linked to a detectable label. In some embodiments, the detectable label is biotin. In some embodiments, the kit further comprises avidin linked to a second detectable label. In some embodiments, the kit further comprises a detectably-labeled secondary antibody that binds the second monoclonal antibody.

Also provided are methods of detecting human pre-Haptoglobin-2 in a sample, the method comprising: contacting the sample with a monoclonal antibody as described above under conditions such that the antibody binds to human pre-Haptoglobin-2, if present in the sample; and detecting the presence, absence, or quantity of binding of the antibody to human pre-Haptoglobin-2 from the sample.

In some embodiments, the monoclonal antibody is linked to a solid support. In some embodiments, after the contacting, unbound components of the sample are washed away from the antibody linked to the solid support while human pre-Haptoglobin-2, if present, remains bound to the antibody, and the method further comprises contacting the human pre-Haptoglobin-2 bound to the antibody linked to the solid support with a second monoclonal antibody that binds human pre-Haptoglobin-2, and wherein the detecting comprises detecting the presence, absence, or quantity of the second monoclonal antibody.

In some embodiments, the antibody is detectably-labeled and the detecting comprises detecting the detectably-labeled antibody with a microscope.

Also disclosed herein is a sterile composition suitable for administration to an animal comprising a non-full length fragment of pre-Haptoglobin-2 comprising or consisting of GYVEHSVRY (SEQ ID NO:1). The fragment may comprise or consist of KPPEIAHGYVEHSVRYQCKNYYK (SEQ ID NO:2). The fragment may comprise or consist of PPEIAHGYVEHSVRY (SEQ ID NO:4), PEIAHGYVEHSVRYQ (SEQ ID NO:5), EIAHGYVEHSVRYQC (SEQ ID NO:6), IAHGYVEHSVRYQCK (SEQ ID NO:7), AHGYVEHSVRYQCKN (SEQ ID NO:8), HGYVEHSVRYQCKNY (SEQ ID NO:9), GYVEHSVRYQCKNYY (SEQ ID NO:10), or YVEHSVRYQCKNYYK (SEQ ID NO:11). The fragment of pre-Haptoglobin-2 may comprises GYVEHSVRY (SEQ ID NO:1) and no more than 20, 15, 10, or 5 adjacent amino acids from pre-Haptoglobin-2. The fragment of pre-Haptoglobin-2 may comprise at least one heterologous amino acid, i.e., at least one amino acid, optionally at an end of the fragment, that does not occur at that position in the native pre-Haptoglobin-2 protein. The sterile composition may further comprise one or more carrier, excipient or adjuvant. Adjuvants include, for example, aluminum hydroxide, lipid A, killed bacteria, polysaccharide, mineral oil, Freund's incomplete adjuvant, Freund's complete adjuvant, aluminum phosphate, iron, zinc, a calcium salt, acylated tyrosine, an acylated sugar, a CpG oligonucleotide, a cationically derivatized polysaccharide, an anionically derivatized polysaccharide, a polyphosphazine, a biodegradable microsphere, a monophosphoryl lipid A, MF59, oil in water emulsions AS03 and AS04, ISCOM, and quil A.

Also disclosed herein is a method of raising antibodies that bind pre-Haptoglobin-2 (and optionally have the other binding properties of the antibodies described herein), the method comprising administering the sterile composition as described above to an animal, and selecting antibodies that bind to pre-Haptoglobin-2.

### BRIEF DESCRIPTION OF THE DRAWINGS

Figure 1 provides a table of data summarizing results of binding data for two different antibody pairs (13D11+1 1G3-bt and 9G7+16H4-bt) using a sandwich ELISA as described in the Example.

Figure 2 provides data showing the ability of the antibody pairs to detect pre-Haptoglobin-2 in the presence of different Haptoglobin forms at known physiological levels.

Figure 3 provides K_{D} (Kₐ/K_{d}) data for various antibodies as determined by surface plasmon resonance (SPR).

Figure 4 provides epitope mapping data, specifically a dot blot showing peptide binding for antibodies. The peptides represent overlapping peptides covering the entire sequences of pre-Haptoglobin-2.

Figure 5 shows that under native (no SDS), non-reducing conditions, all antibodies were capable of recognizing pre-Haptoglobin-1 and pre-Haptoglobin 2, but not mature (processed) haptoglobins. Reduced proteins run under denaturing (SDS) conditions, on the other hand, were only recognized by 13D11.

Figure 6 shows specificity and sensitivity data for antibody pair 13D11+11G3-bt using a sandwich ELISA on human serum.

Figure 7 shows data from the Alpco Zonulin ELISA and the antibody pair 13D11+1 1G3-bt on normal and celiac patient samples.

### DEFINITIONS

As used herein, an "antibody" refers to a protein functionally defined as a binding protein and structurally defined as comprising an amino acid sequence that is recognized by one of skill as being derived from the framework region of an immunoglobulin-encoding gene of an animal that produces antibodies. An antibody can consist of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains, respectively.

The term antibody as used herein includes antibody fragments that retain binding specificity. For example, there are a number of well characterized antibody fragments. Thus, for example, pepsin digests an antibody C-terminal to the disulfide linkages in the hinge region to produce F(ab)'2, a dimer of Fab which itself is a light chain joined to VH-CH1 (Fd) by a disulfide bond. The F(ab)'2 may be reduced under mild conditions to break the disulfide linkage in the hinge region thereby converting the (Fab')2 dimer into an Fab' monomer. The Fab' monomer is essentially a Fab with all or part of the hinge region (see, Fundamental Immunology, W.E. Paul, ed., Raven Press, N.Y. (1993), for a more detailed description of other antibody fragments). While various antibody fragments are defined in terms of the digestion of an intact antibody, one of skill will appreciate that fragments can be synthesized de novo either chemically or by utilizing recombinant DNA methodology. Thus, the term "antibody" also includes antibody fragments produced either by the modification of whole antibodies or synthesized using recombinant DNA methodologies. Antibodies include dimers such as V_{H}-V_{L} dimers, V_{H} dimers, or V_{L} dimers, including single chain antibodies. Alternatively, the antibody can be another fragment, such as a disulfide-stabilized Fv (dsFv). Other fragments can also be generated using known techniques, including using recombinant techniques. Antibodies may include those that have been displayed on phage or generated by recombinant technology using vectors where the chains are secreted as soluble proteins, e.g., scFv, Fv, Fab, (Fab')2 or generated by recombinant technology using vectors where the chains are secreted as soluble proteins.

A "monoclonal antibody" refers to an antibody generated from a clonal antibody-producing cell such as a hybridoma, lymphocyte, or a recombinant antibody-producing cell. "Clonal" means the cells have been cultured in a pure culture in the absence of other cells. Monoclonal antibodies can be prepared using hybridoma methods, such as those described by Kohler & Milstein, Nature 256:495 (1975). In a hybridoma method, a mouse, rat, rabbit, or other appropriate host animal, is typically immunized with an immunizing agent to elicit lymphocytes that produce or are capable of producing antibodies that will specifically bind to the immunizing agent. Alternatively, the lymphocytes may be immunized *in vitro.* The resulting lymphocytes are fused with myeloma or other tumor cells to generate hybridoma cells capable of repeated cell divisions. Clonal hybridomas can then be selected, e.g., via dilution or single-cell selection.

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of epitope mapping are well known in the art (see, *e.g.,* Epitope Mapping Protocols in Methods in Molecular Biology, Vol. 66, Glenn E. Morris, Ed (1996)).

The phrase "specifically (or selectively) binds" to an antibody or "specifically (or selectively) immunoreactive with," when referring to a protein or peptide, refers to a binding reaction where the antibody (or an antibody pair in a sandwich immunoassay) binds to the protein of interest (the antigen). In the context of this application, an antibody pair that specifically detects pre-Haptoglobin-2 typically binds to pre-Haptoglobin-2 with a reactivity that is at least 5 or 10-fold better than the reactivity of the same antibody pair for Haptoglobin-2, pre-Haptoglobin-1, or Haptoglobin-1.

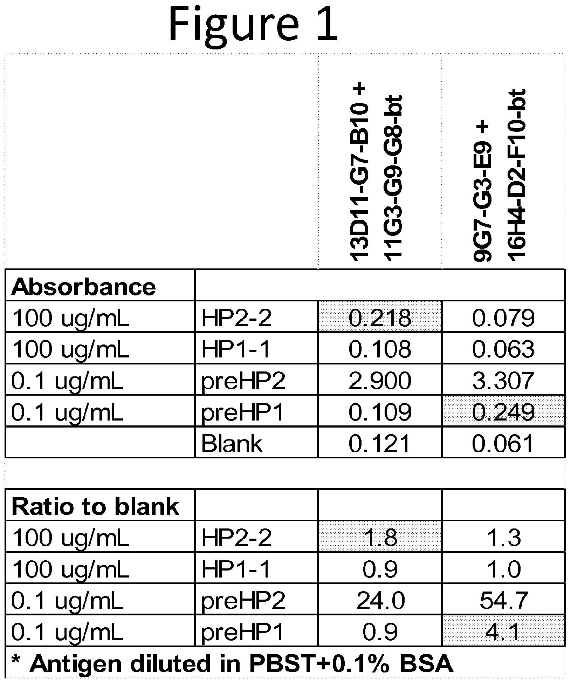

As used herein, a pair of antibodies does not "specifically bind" to a non-target protein if the pair of antibodies bind to pre-Haptoglobin-2 with a reactivity at least 10-fold greater than the reactivity for the non-target protein. For example, while antibody pairs 13D11+11G3 or 9G7+16H4 weakly bind to Haptoglobin-2 and pre-Haptoglobin-1, respectively, these pairs bind to pre-Haptoglobin-2 with an reactivity at least 10-fold higher (see Figure 1), and thus the antibody pairs are not considered to "significantly bind" to Haptoglobin-2 or pre-Hapotoglobin-1.

A "label" or a "detectable moiety" is a heterologous composition detectable by spectroscopic, photochemical, biochemical, immunochemical, chemical, or other physical means. For example, useful labels include fluorescent dyes, electron-dense reagents, enzymes (*e.g.,* as commonly used in an ELISA), radioactive labels, biotin, digoxigenin, or haptens and proteins or other entities which can be made detectable. The labels may be incorporated into the antibodies at any position. Moreover the labels need not be directly conjugated to the antibody, but can be present on a secondary detection agents, such as a secondary antibody that binds the anti- pre-Haptoglobin-2 antibody. Any method known in the art for conjugating the antibody to the label may be employed, e.g., using methods described in Hermanson, Bioconjugate Techniques 1996, Academic Press, Inc., San Diego.

An antibody light or heavy chain variable region consists of a "framework" region interrupted by three hypervariable regions, also called complementarity-determining regions or CDRs. The extent of the framework region and CDRs have been defined (see, "Sequences of Proteins of Immunological Interest," E. Kabat, et al., U.S. Department of Health and Human Services, (1987)). The sequences of the framework regions of different light or heavy chains are relatively conserved within a species. The framework region of an antibody, that is the combined framework regions of the constituent light and heavy chains, serves to position and align the CDRs in three dimensional space. The CDRs are primarily responsible for binding to an epitope of an antigen. The CDRs are typically referred to as CDR1, CDR2, and CDR3, numbered sequentially starting from the N-terminus.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor or drug; or (b) the variable region, or a portion thereof, is altered, replaced or exchanged with a variable region having a different or altered antigen specificity.

"Complementarity-determining region" or "CDR", also generally known as hypervariable regions or hypervariable loops, refers to the art-recognized term as exemplified by Chothia and Lesk (1987) J. Mol. Biol. 196: 901; Chothia et al. (1989) Nature 342: 877; Kabat et al., Sequences of Proteins of Immunological Interest (National Institutes of Health, Bethesda, Md.) (1987); and Tramontano et al. (1990) J. Mol. Biol. 215: 175. "Framework region of the variable region" or "FR" refers to the region of the V domain that flank the CDRs. The positions of the CDRs and framework regions can be determined using various well known definitions in the art as described in, *e.g.,* Kabat, *supra,* Chothia, *supra,* international ImMunoGeneTics database (IMGT), and AbM (*see, e.g.,* Chothia & Lesk, 1987, J. Mol. Biol. 196, 901-917; Chothia, et al., 1989, Nature 342, 877-883; Chothia, et al., 1992, J. Mol. Biol. 227, 799-817; Al-Lazikani et al., J.Mol.Biol 1997, 273(4)). Definitions of CDRs are also described in the following: Ruiz et al., IMGT, the international ImMunoGeneTics database. Nucleic Acids Res., 28, 219-221 (2000); and Lefranc,M.-P. IMGT, the international ImMunoGeneTics database. Nucleic Acids Res. Jan 1;29(1):207-9 (2001); MacCallum et al, J. Mol. Biol., 262 (5), 732-745 (1996); Martin et al, Proc. Natl Acad. Sci. USA, 86, 9268-9272 (1989); Martin, et al, Methods Enzymol., 203, 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

### DETAILED DESCRIPTION OF THE INVENTION

### I. Introduction

Disclosed herein are monoclonal antibodies that have high affinity for human pre-Haptoglobin-2. Moreover, some antibody pairs have high reactivity for human pre-Haptoglobin-2, i.e., the antibody pairs detect human pre-Haptoglobin-2 but do not detect, or do not significantly detect, human pre-Haptoglobin-1, human Haptoglobin-1, or human Haptoglobin-2. Tripathi, et al. published findings suggesting that zonulin is pre-haptoglobin 2 (PNAS 106(39): 16799-804, 2009). As discussed in the Examples, the inventors have surprisingly found that these antibody pairs specifically detect pre-Haptoglobin-2 with much better specificity than commercially-available polyclonal antibodies used in an ELISA sold as specific for Zonulin. The herein disclosed pairs of antibodies that allow for specific detection of pre-Haptoglobin-2, even in the presence of human pre-Haptoglobin-1, human Haptoglobin-1, or human Haptoglobin-2. Surprisingly, when some of the individual monoclonal antibodies are tested, they bind at least one of human pre-Haptoglobin-1, human Haptoglobin-1, or human Haptoglobin-2 in addition to human pre-Haptoglobin-2. However, when specific pairs of antibodies were tested in a sandwich immunoassay, the pairs specifically detect human pre-Haptoglobin-2 and do not significantly detect human pre-Haptoglobin-1, human Haptoglobin-1, or human Haptoglobin-2.

### II. Antibodies specific for Pre-Haptoglobin-2

Provided herein are monoclonal antibodies that bind to human pre-Haptoglobin-2 but do not bind human pre-Haptoglobin-1, human Haptoglobin-1, or human Haptoglobin-2 (e.g., as determined by surface plasmon resonance). These monoclonal antibodies bind to a number of overlapping peptides comprising GYVEHSVRY (SEQ ID NO:1). In some embodiments, the monoclonal antibodies having the above criteria have a K_{D} for human pre-Haptoglobin-2 of less than 25 or 10 nM, e.g., 25-1 or 10-1 nM (e.g., as determined by surface plasmon resonance). As known in the art, a lower K_{D} value indicates stronger binding (higher affinity) to the target.

In additional embodiments, these monoclonal antibodies bind to native (lacking a denaturant), non-reduced (lacking a reducing agent (e.g. β-mercaptoethanol (BME) or dithiothreitol)), human pre-Haptoglobin-2, but do not significantly bind reduced, denatured human pre-Haptoglobin-2. Thus, a sample comprising pre-Haptoglobin-2 run on a gel with a denaturant (e.g.,SDS) and a reducing agent (e.g., BME) and blotted on a western blot (e.g., a typical western blotting procedure) represents a reduced, denatured pre-Haptoglobin-2. A sample comprising pre-Haptoglobin-2 run on a gel lacking a denaturant (e.g., lacking SDS) and lacking a reducing agent (e.g., lacking BME) and blotted on a western blot represents a native, non-reduced pre-Haptoglobin-2. Generally, reducing conditions are thought to disrupt secondary structure of proteins and thus it is believed that the monoclonal antibodies that bind non-reduced human pre-Haptoglobin-2, but do not significantly bind reduced human pre-Haptoglobin-2, bind to three-dimensional (non-linear) epitopes on human pre-Haptoglobin-2. In some embodiments, the monoclonal antibodies having the criteria described in this paragraph have a K_{D} for human pre-Haptoglobin-2 of less than 25or 10 nM, e.g., 25-0.1 or 10-0.1 nM.

Antibodies described herein can be generated as desired. Monoclonal antibodies may be prepared using hybridoma methods. A mouse, hamster, or other appropriate host animal may be immunized with an immunizing agent to elicit lymphocytes that produce or are capable of producing antibodies that will specifically bind to the immunizing agent. Alternatively, the lymphocytes may be immunized *in vitro.* An appropriate immunogen can be, for example, a recombinant full-length pre-Haptoglobin-2 (e.g. affinity-tagged and expressed by recombinant insect cells). A variety of methods are known and can be used for generating hybridomas or other antibody-producing cells. *See,* e.g., Harlow, ANTIBODIES, Cold Spring Harbor Press, N.Y. (1989). To generate hybridomas producing monoclonal antibodies to an antigen, splenocytes and lymph node cells from immunized animals can be isolated and fused to an appropriate immortalized cell line, such as a mouse myeloma cell line. The resulting hybridomas can then be screened for the production of antigen-specific antibodies. For example, single cell suspensions of splenic lymphocytes from immunized mice can be fused to SP2/0-Ag14 myeloma cells (ATCC, CRL 1581) with 1 ml PEG1500 (Roche). Cells can be plated at approximately 3x10⁴ per well in flat bottom microtiter plate, followed by a two-week incubation in selective medium containing besides usual reagents 20 % Hyclone Fetal Bovine Serum, 10 % Hybridoma Cloning Supplement (PAA), 1 % OPI Media Supplement (Sigma) and 2 % Azaserine-Hypoxanthine (Sigma). A plurality of hybridoma culture supernatants (or other antibody solutions) can be screened in parallel for desired binding properties.

It may be desirable to use human monoclonal antibodies. Human monoclonal antibodies can be produced using various techniques known in the art, including phage display libraries. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, e.g., in U.S. Patents. 5,545,807; 5,545,806; 5,569,825; 5,625,126; 5,633,425; 5,661,016, and in the following scientific publications: Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); Lonberg & Huszar, Intern. Rev. Immunol. 13:65-93 (1995).

A variety of screening assays for identifying hybridomas with the desired reactivity will be clear based on the disclosure and Examples presented herein. As an example, recombinant (or purified) preHP2, preHP1, HP1, and HP2 can be coated to plates and the hybridoma supernatant can then be applied to the plates. Detection of binding can be achieved using an anti-mouse antibodies linked to horseradish peroxidase (HRP) (a direct ELISA format). Second, the plates can be coated with the hybridoma supernatant via an anti-mouse antibody, protein can be applied, and binding then detected using a pan-haptoglobin antibody followed by contact with secondary antibodies linked to HRP (a sandwich ELISA format).

Any of the antibodies described herein can be isolated from other biological components, e.g., can be in a solution separate from cells or cellular components. The antibodies may represent at least 90, 95, or 99 % of all protein in the solution.

In some embodiments, one or more antibody described herein can be linked to a solid support. Any type of solid support can be used as desired. The solid support can be the wall or floor of an assay vessel, or a dipstick or other implement to be inserted into an assay vessel, or particles placed inside or suspended in an assay vessel. Particles, and especially beads, are particularly useful in many embodiments, including beads that are microscopic in size (i.e., microparticles) and formed of a polymeric material. Polymers useful as microparticles are those that are chemically inert relative to the components of the biological sample and to the assay reagents other than the binding members that are immobilized on the microparticle surface. Exemplary microparticle materials, particularly when fluorescent labels are used in the assay, are those with minimal autofluorescence, and that are solid and insoluble in the sample and in any buffers, solvents, carriers, diluents, or suspending agents used in the assay, in addition to allowing immobilization of the assay reagent. Examples of suitable polymers are polystyrenes, polyesters, polyethers, polyolefins, polyalkylene oxides, polyamides, polyurethanes, polysaccharides, celluloses, and polyisoprenes. Crosslinking is useful in many polymers for imparting structural integrity and rigidity to the microparticle. The size range of the microparticles can vary. The microparticles may range in diameter from 0.3 µm to 100 µm, and in other cases, from 0.5 µm to µm 40 µm, and in still other cases, from 2 µm to 10 µm.

Attachment of the antibodies to the surfaces of the solid support can be achieved, for example, by electrostatic attraction, specific affinity interaction, hydrophobic interaction, or covalent bonding. Functional groups for covalent bonding can be incorporated into the polymer structure by conventional means, such as the use of monomers that contain the functional groups, either as the sole monomer or as a co-monomer. Examples of suitable functional groups are amine groups (-NH₂), ammonium groups (-NH₃⁺ or -NR₃⁺), hydroxyl groups (-OH), carboxylic acid groups (-COOH), and isocyanate groups (-NCO). Useful monomers for introducing carboxylic acid groups into polyolefins, for example, are acrylic acid and methacrylic acid. Linking groups can also be used for increasing the density of the antibodies on the solid phase surface and for decreasing steric hindrance to increase the range and sensitivity of the assay. Examples of suitable useful linking groups are polylysine, polyaspartic acid, polyglutamic acid and polyarginine.

In some embodiments, one or more antibody described herein can be linked to a label. The particular label or detectable group used is not critical, as long as it does not significantly interfere with the specific binding of the antibody used and allows for detection of the antibody. The label can be directly attached to the antibody or to another agent used in the detection assay, such as a secondary antibody. The detectable group can be any material having a detectable physical or chemical property. Such detectable labels have been well-developed in the field of immunoassays and, in general, any label useful in such methods can be applied to the antibodies described here. A label is any composition directly or indirectly detectable by spectroscopic, photochemical, biochemical, immunochemical, electrical, optical or chemical means. Useful labels include fluorescent compounds (e.g., fluorescein isothiocyanate, Texas red and rhodamine, fluorescein), radiolabels, enzymes (e.g., horse radish peroxidase, alkaline phosphatase and others commonly used in an ELISA), streptavidin/biotin, and colorimetric labels such as colloidal gold or colored glass or plastic beads (e.g., polystyrene, polypropylene or latex). Chemiluminescent compounds may also be used.

One or more anti-pre-Haptiglobin-2 antibody disclosed herein may have the CDRs (i.e., CDRs 1, 2, and 3 from the heavy chain variable region and CDRs1, 2, and 3 from the light chain variable region) from one of: 13D11, 11G3, 9G7, and 16H4. Once a CDR is identified from 13D11, 11G3, 9G7, or 16H4, the CDRs, or the entire variable regions, can be cloned back into an immunoglobulin scaffold. Examples of this technique include the CDR grafting antibody technique. *See, e.g.,* P.T. Jones et al., Nature, 321:522, 1986. In some cases, the region comprising the selected CDRs and the immunoglobulin scaffolds in which the CDRs are inserted are from the same animal. The CDRs and scaffold may also be from different animals. cDNAs encoding the variable regions thus constructed may be expressed as desired. Cells, e.g. mammalian cells, comprising vectors for expressing the cDNAs can also be provided.

In other cases, the CDRs are used to make a single chain antibody (scFv). To create a scFv gene, the VH- and VL-encoding DNA fragments are operatively linked to another fragment encoding a flexible linker such that the VH and VL sequences can be expressed as a contiguous single-chain protein, with the VL and VH regions joined by the flexible linker (*see e.g.,* Bird et al. (1988) Science 242:423-426; Huston et al. (1988) Proc. Natl. Acad. Sci. USA 85:5879-5883; McCafferty et al., Nature (1990) 348:552-554).

### III. Methods of detecting Pre-Haptoglobin-2

A variety of immunoassay techniques, including competitive and non-competitive immunoassays, employing one or more (e.g., two) antibodies having the binding properties as described herein, can be used to detect the presence, absence, or level of pre-Haptoglobin-2 in a sample. A variety of immunoassay formats are described in, e.g., Self and Cook, Curr. Opin. Biotechnol., 7:60-65 (1996)). The term immunoassay encompasses techniques including enzyme immunoassays (EIA) such as enzyme multiplied immunoassay technique (EMIT), enzyme-linked immunosorbent assay (ELISA), antigen capture ELISA, sandwich ELISA, IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); and chemiluminescence assays (CL). If desired, such immunoassays can be automated. Immunoassays can also be used in conjunction with laser induced fluorescence (see, e.g., Schmalzing and Nashabeh, Electrophoresis, 18:2184-2193 (1997); Bao, J. Chromatogr. B. Biomed. Sci., 699:463-480 (1997).

Antigen capture ELISA can be useful for detecting the presence or level of pre-Haptoglobin-2 in a sample. For example, in an antigen capture ELISA, an antibody directed to pre-Haptoglobin-2 is linked to a solid phase and sample is added such that pre-Haptoglobin-2, if present in a sample, is bound by the antibody. After unbound proteins are removed by washing, the amount of bound marker can be quantitated using, e.g., a radioimmunoassay (see, e.g., Harlow and Lane, Antibodies: A Laboratory Manual, Cold Spring Harbor Laboratory, New York, 1988)).

As explained in the examples, sandwich ELISA immunoassays are also suitable for use in detecting pre-Haptoglobin-2. For example, in a two-antibody sandwich assay, a first (capture) antibody is bound to a solid support, and pre-Haptoglobin-2, if present in the sample, is allowed to bind to the first antibody. In some embodiments, other components of the sample are removed (e.g., washed away) before a second (detection) antibody is contacted to the antigen bound to the capture antibody. The amount of the marker is quantitated by measuring the amount of a second (capture) antibody that binds pre-Haptoglobin-2. The antibodies can be immobilized onto a variety of solid supports, such as magnetic or chromatographic matrix particles, the surface of an assay plate (e.g., microtiter wells) and pieces of a solid substrate material or membrane (e.g., plastic, nylon, paper) as described herein. An assay strip may be prepared by coating the antibody or a plurality of antibodies in an array on a solid support. This strip can then be dipped into the test sample and processed quickly through washes and detection steps to generate a measurable signal, such as a colored spot. The capture antibody binds to GYVEHSVRY (SEQ ID NO:1), or a fragment of pre-Haptoglobin-2 comprising GYVEHSVRY (SEQ ID NO:1). The monoclonal antibody may bind KPPEIAHGYVEHSVRYQCKNYYK (SEQ ID NO:2). The monoclonal antibody may bind KPPEIAHGYVEHSVR (SEQ ID NO:3), PPEIAHGYVEHSVRY (SEQ ID NO:4), PEIAHGYVEHSVRYQ (SEQ ID NO:5), EIAHGYVEHSVRYQC (SEQ ID NO:6), IAHGYVEHSVRYQCK (SEQ ID NO:7), AHGYVEHSVRYQCKN (SEQ ID NO:8), HGYVEHSVRYQCKNY (SEQ ID NO:9), GYVEHSVRYQCKNYY (SEQ ID NO:10), and/or YVEHSVRYQCKNYYK (SEQ ID NO:11). The monoclonal antibody may bind one or more peptide listed above but may not bind PKPPEIAHGYVEHSV (SEQ ID NO:12) or VEHSVRYQCKNYYKL (SEQ ID NO:13).

The capture antibody may bind non-reduced pre-Haptoglobin-2 but may not bind to reduced pre-Haptoglobin-2. The detection antibody binds to GYVEHSVRY (SEQ ID NO:1), or a fragment of pre-Haptoglobin-2 comprising GYVEHSVRY (SEQ ID NO:1). The monoclonal antibody may bind KPPEIAHGYVEHSVRYQCKNYYK (SEQ ID NO:2). The monoclonal antibody may bind KPPEIAHGYVEHSVR (SEQ ID NO:3), PPEIAHGYVEHSVRY (SEQ ID NO:4), PEIAHGYVEHSVRYQ (SEQ ID NO:5), EIAHGYVEHSVRYQC (SEQ ID NO:6), IAHGYVEHSVRYQCK (SEQ ID NO:7), AHGYVEHSVRYQCKN (SEQ ID NO:8), HGYVEHSVRYQCKNY (SEQ ID NO:9), GYVEHSVRYQCKNYY (SEQ ID NO:10), and/or YVEHSVRYQCKNYYK (SEQ ID NO:11). The monoclonal antibody may bind one or more peptide listed above but may not bind PKPPEIAHGYVEHSV (SEQ ID NO:12) or VEHSVRYQCKNYYKL (SEQ ID NO:13). In some embodiments, the detection antibody binds non-reduced pre-Haptoglobin-2 but does not bind to reduced pre-Haptoglobin-2. As discussed elsewhere herein, One or both antibodies of the pair may have affinity for Haptoglobin-2 or pre-Haptoglobin-1 as well as for pre- Haptoglobin -2. Nevertheless, when paired in a sandwich assay, the antibody pair specifically detects pre-Haptoglobin-2 and does not significantly detect Haptoglobin-2 or pre-Haptoglobin-1. The antibody pair may detect pre-Hapoglobin-2 with better sensitivity and specificity than a sandwich assay based on polyclonal antibodies directed to Zonulin.

A radioimmunoassay using, for example, an iodine-125 (¹²⁵I) labeled secondary antibody (Harlow and Lane, *supra*) is also suitable for detecting the presence or level of one or more markers in a sample. A secondary antibody labeled with a chemiluminescent marker can also be suitable. A chemiluminescence assay using a chemiluminescent secondary antibody is suitable for sensitive, non-radioactive detection of marker levels. Such secondary antibodies can be obtained commercially from various sources, e.g., Amersham Lifesciences, Inc. (Arlington Heights, 111.).

Specific immunological binding of the antibody to pre-Haptoglobin-2 can be detected directly or indirectly. Direct labels include fluorescent or luminescent tags, metals, dyes and radionuclides, attached to the antibody. An antibody labeled with iodine-125 (¹²⁵I) can be used for determining the level of pre-Haptoglobin-2 in a sample. A chemiluminescence assay using a chemiluminescent antibody specific for pre-Haptoglobin-2 is suitable for sensitive, non-radioactive detection of pre-Haptoglobin-2 levels. An antibody labeled with fluorochrome is also suitable for determining the levels of pre-Haptoglobin-2 in a sample. Examples of fluorochromes include DAPI, fluorescein, Hoechst 33258, R-phycocyanin, B-phycoerythrin, R-phycoerythrin, rhodamine, Texas red, and lissamine. Secondary antibodies linked to fluorochromes can be obtained commercially, e.g., goat F(ab')₂ anti-human IgG-FITC is available from Tago Immunologicals (Burlingame, Calif.).

Indirect labels include various enzymes well-known in the art, such as horseradish peroxidase (HRP), alkaline phosphatase (AP), β-galactosidase and urease. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β galactosidase detection system can be used with the chromogenic substrate o-nitrophenyl- β-D-galactopyranoside (ONPG), which yields a soluble product detectable at 410 nm. A urease detection system can be used with a substrate such as urea-bromocresol purple (Sigma Immunochemicals; St. Louis, Mo.). A useful secondary antibody linked to an enzyme can be obtained from a number of commercial sources, e.g., goat F(ab')₂ anti-human IgG-alkaline phosphatase can be purchased from Jackson ImmunoResearch (West Grove, Pa.).

A signal from the direct or indirect label can be analyzed, for example, using a spectrophotometer to detect color from a chromogenic substrate; a radiation counter to detect radiation such as a gamma counter for detection of ¹²⁵I; or a fluorometer to detect fluorescence in the presence of light of a certain wavelength. For detection of enzyme-linked antibodies, a quantitative analysis of the amount of marker levels can be made using a spectrophotometer such as an EMAX Microplate Reader (Molecular Devices; Menlo Park, Calif.) in accordance with the manufacturer's instructions. If desired, the assays described herein can be automated or performed robotically, and the signal from multiple samples can be detected simultaneously.

Quantitative Western blotting can also be used to detect or determine the presence or level of pre-Haptoglobin-2 in a sample. Western blots can be quantitated by methods such as scanning densitometry or phosphorimaging. As an, protein samples are electrophoresed on 10 % SDS-PAGE Laemmli gels. Murine monoclonal antibodies are reacted with the blot, and antibody binding can be confirmed to be linear using a preliminary slot blot experiment. Goat anti-mouse horseradish peroxidase-coupled antibodies (BioRad) are used as the secondary antibody, and signal detection performed using chemiluminescence, for example, with the Renaissance chemiluminescence kit (New England Nuclear; Boston, Mass.) according to the manufacturer's instructions. Autoradiographs of the blots are analyzed using a scanning densitometer (Molecular Dynamics; Sunnyvale, Calif.) and normalized to a positive control. Values are reported, for example, as a ratio between the actual value to the positive control (densitometric index). Such methods are well known in the art as described, for example, in Parra et al., J. Vasc. Surg., 28:669-675 (1998).

Alternatively, a variety of immunohistochemical assay techniques can be used to detect or determine the presence or level of pre-Haptoglobin-2 in a sample. The term "immunohistochemical assay" encompasses techniques that utilize the visual detection of fluorescent dyes or enzymes coupled (i.e., conjugated) to antibodies that react with pre-Haptoglobin-2 using fluorescent microscopy or light microscopy (e.g., in a tissue slice) and includes direct fluorescent antibody assay, indirect fluorescent antibody (IFA) assay, anticomplement immunofluorescence, avidin-biotin immunofluorescence, and immunoperoxidase assays. An IFA assay, for example, is useful for determining whether a sample is positive for pre-Haptoglobin-2 or the level of pre-Haptoglobin-2 in a sample. The concentration of pre-Haptoglobin-2 in a sample can be quantitated, e.g., through endpoint titration or through measuring the visual intensity of fluorescence compared to a known reference standard.

Pre-Haptoglobin-2 may be detected as part of a multiplex assay. The analysis of a plurality of markers may be carried out separately or simultaneously with one test sample. For separate or sequential assay of markers, suitable apparatuses include clinical laboratory analyzers such as the ElecSys (Roche), the AxSym (Abbott), the Access (Beckman), the ADVIA™, the CENTAUR™ (Bayer), and the NICHOLS ADVANTAGE™ (Nichols Institute) immunoassay systems. Exemplary apparatuses or protein chips perform simultaneous assays of a plurality of markers on a single surface. Exemplary physical formats comprise surfaces having a plurality of discrete, addressable locations for the detection of a plurality of different markers. Such formats include protein microarrays, or "protein chips" (see, e.g., Ng et al., J. Cell Mol. Med., 6:329-340 (2002)) and certain capillary devices (*see, e.g.,* U.S. Pat. No. 6,019,944). In these cases, each discrete surface location may comprise antibodies to immobilize one or more markers for detection at each location. Surfaces may alternatively comprise one or more discrete particles (e.g., microparticles or nanoparticles) immobilized at discrete locations of a surface, where the microparticles comprise antibodies to immobilize one or more markers for detection.

Detection of pre-Haptoglobin-2 can be used for providing a diagnosing or prognosis, monitoring disease and/or monitoring treatment of autoimmune and inflammatory disease or allergies. Expression of pre-Haptoglobin-2 is associated with autoimmune and inflammatory diseases as well as allergies. For example, expression of pre-Haptoglobin-2 is associated with celiac disease and Type-1 diabetes. *See, e.g.,* US Patent Publication 2012/0107329.

### IV. Kits

Also provided are kits for performing an immunoassay employing one or more (e.g., two) pre-Haptoglobin-2 antibody as described herein. The kit comprises a least one pre-Haptoglobin-2 antibody as described herein linked to a solid support. In some embodiments, the kit comprises at least one pre-Haptoglobin-2 antibody as described herein linked to a label. In some embodiments, the kit comprises a least one pre-Haptoglobin-2 antibody as described herein linked to a solid support and at least one pre-Haptoglobin-2 antibody as described herein linked to a label. The antibody linked to the solid support binds to GYVEHSVRY (SEQ ID NO:1), or a fragment of pre-Haptoglobin-2 comprising GYVEHSVRY (SEQ ID NO:1). The antibody linked to the solid support may bind KPPEIAHGYVEHSVRYQCKNYYK (SEQ ID NO:2). The antibody linked to the solid support may bind KPPEIAHGYVEHSVR (SEQ ID NO:3), PPEIAHGYVEHSVRY (SEQ ID NO:4), PEIAHGYVEHSVRYQ (SEQ ID NO:5), EIAHGYVEHSVRYQC (SEQ ID NO:6), IAHGYVEHSVRYQCK (SEQ ID NO:7), AHGYVEHSVRYQCKN (SEQ ID NO:8), HGYVEHSVRYQCKNY (SEQ ID NO:9), GYVEHSVRYQCKNYY (SEQ ID NO:10), and/or YVEHSVRYQCKNYYK (SEQ ID NO:11). The antibody linked to the solid support may bind one or more peptide listed above but may not bind PKPPEIAHGYVEHSV (SEQ ID NO:12) or VEHSVRYQCKNYYKL (SEQ ID NO:13). At least one pre-Haptoglobin-2 antibody in the kit may have a K_{D} for human pre-Haptoglobin-2 of less than 25, 20, 15, 10, 5, or 1 nM, e.g., 25-0.1 or 10-0.1 nM.

In some embodiments, the kit comprises at least one antibody (e.g., linked to a solid support or linked to a label) that binds to non-reduced human pre-Haptoglobin-2, but do not significantly bind reduced human pre-Haptoglobin-2. Such antibodies do not bind to human pre-Haptoglobin-1 (e.g., as determined by surface plasmon resonance).

The kits can also comprise other reagents, e.g., reagents for using or developing an ELISA assay. For instance, in some embodiments, the kit further comprises a secondary antibody that binds to the pre-Haptoglobin-2 detection antibody (i.e., the antibody in a sandwich assay that is not linked to the solid support).

### EXAMPLES

This example describes generation and characterization of antibodies that bind to pre-Haptoglobin-2 and development of the antibodies to specific detection of pre-Haptoglobin-2.

Full-length recombinant pre-Haptoglobin-2 (preHP2) was generated and purified from cells. 10 µg of recombinant human pre-HP2 (GenBank accession no. NP_005134) expressed in insect cells with a C-terminal hexahistidine (SEQ ID NO:14) tag were emulsified in equal ratio with Sigma Adjuvant System or Alum adjuvant (Thermo Scientific Pierce, Rockford, IL). Four 8-week-old CD1 mice (Charles River) were immunized by intraperitoneal injections. Two booster injections were administrated at the same doses at 2-week intervals. Mice were bled 10 d after each boost and serology was controlled by indirect ELISA to select the best responding mouse. A pre-fusion boost was administered 4 d before fusion. Sp2/0Ag14 myeloma cell line (ATCC CRL 1581) was fused with splenocytes from selected immunized mouse according to standard protocols and fusion product was plated in culture microplates for 15 d before primary screening.

For clones' selection, either indirect or sandwich ELISA were used all along the process: from primary screening, confirmation to sub-cloning screenings. Antigens used were recombinant pre-HP2 as specific antigen and recombinant pre-HP1, purified mature haptoglobin 1 and 2 (Sigma Aldrich) and a non-specific His-tagged protein as control antigens.

For sandwich ELISA format, microplates were first coated with a goat anti-mouse IgG antibody (Jackson Immuno Research, West Grove, PA) before adding hybridoma supernatants; antigen binding was detected with a rabbit anti-pan haptoglobin polyclonal antibody (internal source) followed by the addition of a horseradish peroxidase (HRP) conjugated goat anti-rabbit IgG antibody (Biosource, Grand Island, NY). For the indirect ELISA, antibody binding to adsorbed antigens was detected with a HRP-conjugated goat anti-mouse IgG antibody (Sigma). For the two formats, tetramethylbenzidine (TMB) was used as substrate, and after stopping the reaction with H₂SO₄, optical density (OD) was read at 450nm in an Infinite F200 ELISA reader (Tecan, San Jose, CA).

After primary screening, antibody-producing hybridomas were twice sub-cloned and then frozen in liquid nitrogen. Monoclonal antibodies were produced *in vitro* by collecting concentrated supernatants. Purifications were done by affinity chromatography on Protein A Sepharose (GE Healthcare, Piscataway, NJ). The mAbs were isotyped with a mouse isotyping test kit (Roche, Indianapolis, IN) according to the manufacturer's recommendations.

Only 0.18 % of the screened clones were ultimately determined to specifically bind to pre-Haptoglobin.

Sixteen antibodies were tested in pairs in a sandwich assay using pre-Haptoglobin-2 (pre-HP2), pre-Haptoglobin-1 (pre-HPl), Haptoglobin-2 (HP2-2), and Haptoglobin-1 (HP1-1) as antigen at concentrations of 200 ng/ml or 50 ng/ml. Standard sandwich ELISA assay conditions were used as described above except the detection antibody was biotinylated and binding was detected with streptavidin HRP. Thus, capture antibody was coated onto a 96 well plate (Maxisorp, Nunc), antigen (preHP2) was then added, followed by biotinylated detection antibody and streptavidin HRP, with washing with PBS+0.1 % Tween between each step. Color was developed with 3, 3', 5,5 '-tetramethylbenzidine (TMB). Clones were used as both capture and detection.

Two pairs (13D11+1 1G3-bt and 9G7+16H4-bt, where "bt" indicates the antibody was biotinylated for detection purposes) with the best specificity (ratio of specific to non-specific binding) and sensitivity were selected for validation using a sandwich ELISA. In the Figures, the antibodies are sometimes referred to as follows: 11G3 = "11G3-G9-G8"; 13D11 = "13D11-G7-B10"; 9G7 = "9G7-G3-E9"; and 16H4 = "16H4-D2-F10". As shown in Figure 1, absorbance (indicating binding to the antigen presented) for pre-HP2 was at least 30 times higher than for the blank. Concentrations on the left of Figure 1 indicate the concentrations at which the protein was tested. The mature Haptoglobin proteins were tested at 1000-fold higher concentrations than the pre-Haptoglobin proteins. The reason for this is that mature haptoglobins are known to normally be present in human serum around 1-1.5mg/ml. The assay was intended to be run at a 1:10 dilution of human serum, so 100 ug/ml would be the final concentration of mature haptoglobin in the well to test for cross-reaction with the antibodies. A reference range of normal preHP 1 and preHP2 in human serum was not known, so 0.1 ug/ml was chosen because that level falls in the higher range of the standard curve.

13D11+1 1G3-bt had minor cross-reactivity in this sandwich assay with HP2-2, whereas 9G7+16H4-bt had minor cross-reactivity with pre-HP1. The cross-reactivity is considered insignificant binding in that the signal for pre-HP2 was at least 10-fold higher than signal for pre-HP1 and at least 10,000-fold higher than HP2-2 (keeping in mind HP2-2 was loaded at 1,000-fold higher concentrations than pre-HP2).

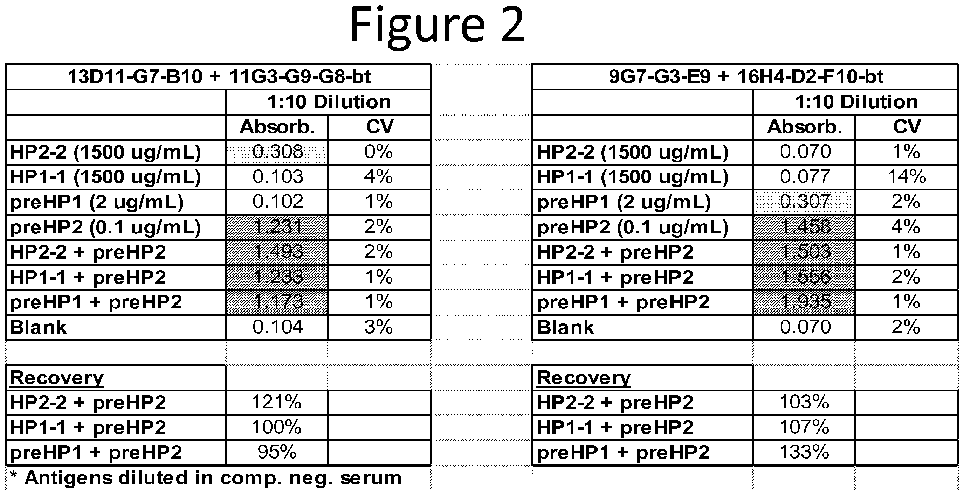

As shown in Figure 2, the ability of the antibody pairs to detect pre-Haptoglobin-2 in the presence of different Haptoglobin forms at known physiological levels was assayed. The presence of other Haptoglobin forms does not interfere with pre-Haptoglobin-2 recovery, but cross-reactivity resulted in a minor additive signal, which was not considered significant in development of the pre-Haptoglobin-2 detection assay. For the data generated for Figure 2, the level of mature HPs was 1.5 mg/ml and of preHP1 was 2 ug/ml. The concentrations in Figure 2 indicate mature HPs and preHP1 concentrations prior to a 1:10 dilution while the preHP2 concentration is listed for after the dilution.

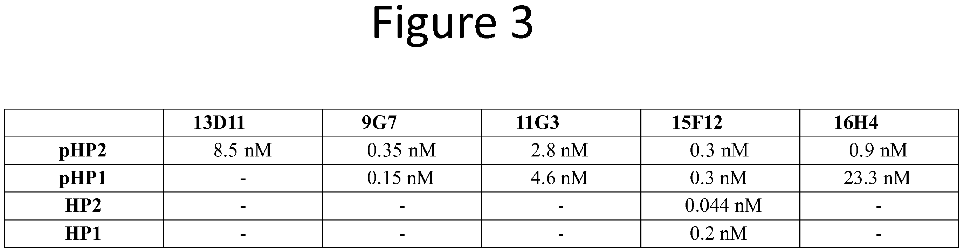

The four antibodies from the test pairs, i.e., 13D11, 11G3, 9G7, and 16H4 were tested by surface plasmon resonance (SPR) for their affinity for pre-Haptoglobin-2. As shown in Figure 3, using SPR, 13D11 displayed only binding affinity for pre-Haptoglobin-2. The remaining three antibodies (11G3, 9G7, and 16H4) had affinity for pre-Haptoglobin-2 as well as pre-Haptoglobin-1. Interestingly, antibody 9G7 displayed a stronger affinity (lower K_{D}) for pre-Haptoglobin-1 than pre-Haptoglobin-2 even though when paired with 16H4, the combination in a sandwich assay specifically detected pre-Haptoglobin-2 with only insignificant pre-Haptoglobin-1 background binding.

Immobilization of the monoclonal antibodies was performed in the vertical orientation of the ProteOn XPR36 system (Bio-Rad) using a flow rate of 30 µl/min at 25 °C on a GLC chip. Four channels were activated for 3 min (90 µl) using a mixture of 20 mM 1-ethyl-3-(3-dimethylaminopropyl) carbodiimide hydrochloride carbodiimide(EDC) and 5 mM sulfo- N-hydroxysulfosuccinimide (sulfo-NHS). This was followed by an immediate injection of 50 µl of 10 ug/ml monoclonal antibody in 10 mM acetate buffer (pH 4.5). Finally, 150 µl of 1 M ethanolamine-HCl (pH 8.5) was injected to deactivate any remaining activated carboxyl groups. This resulted in the immobilization of approximately 1200 to 2000 response units (RU) of the different antibodies in the channels.

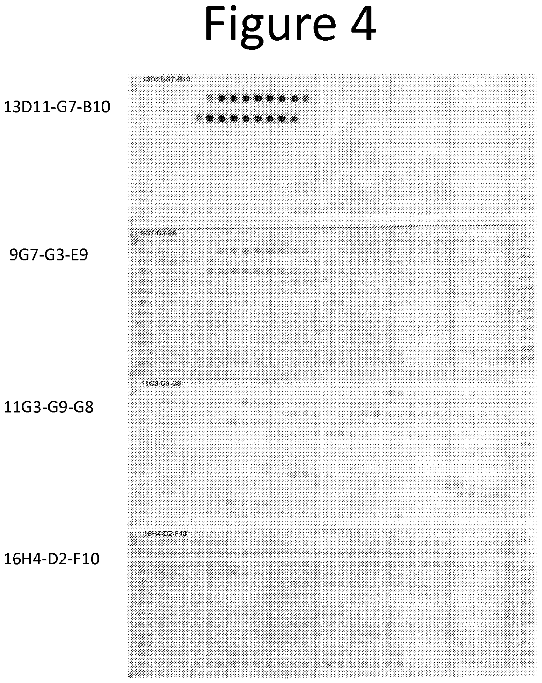

An experiment was performed to determine whether the antibodies bind linear epitopes of pre-Haptoglobin-2. Thus, a series of 15-mer peptides representing peptides along the length of pre-Haptoglobin-2 were spotted on a nitrocellulose membrane and then probed with each of the four antibodies. Interestingly, for three of the antibodies (11G3, 9G7, and 16H4), no signal was detected of any peptide, suggesting that they do not bind a linear epitope of pre-Haptoglobin-2. For the fourth antibody (13D11), a series of overlapping peptides were detected (see Figure 4).

The human pre-Haptoglobin-2 amino acid sequence is provided below (SEQ ID NO:15): Italics: Signal sequence
Bold: α-chain repeat sequence
Underline: 13D11 minimal epitopes

Antibody from hybridoma 13D11 bound the following peptides:

| | |
|---|---|
| 35,94 | KPPEIAHGYVEHSVR (SEQ ID NO:3) |
| 36,95 | PPEIAHGYVEHSVRY (SEQ ID NO:4) |
| 37,96 | PEIAHGYVEHSVRYQ (SEQ ID NO:5) |
| 38,97 | EIAHGYVEHSVRYQC (SEQ ID NO:6) |
| 39,98 | IAHGYVEHSVRYQCK (SEQ ID NO:7) |
| 40,99 | AHGYVEHSVRYQCKN (SEQ ID NO:8) |
| 41,100 | HGYVEHSVRYQCKNY (SEQ ID NO:9) |
| 42,101 | GYVEHSVRYQCKNYY (SEQ ID NO:10) |
| 43,102 | YVEHSVRYQCKNYYK (SEQ ID NO:1 1). |

Antibody from hybridoma 13D11 did not bind the following peptides, which are adjacent to those listed above:
34, 93 : PKPPEIAHGYVEHSV (SEQ ID NO:12)
44, 103: VEHSVRYQCKNYYKL (SEQ ID NO:13).

Based on this data, it was determined that 13D11 binds to the minimal epitope GYVEHSVRY (SEQ ID NO:1).

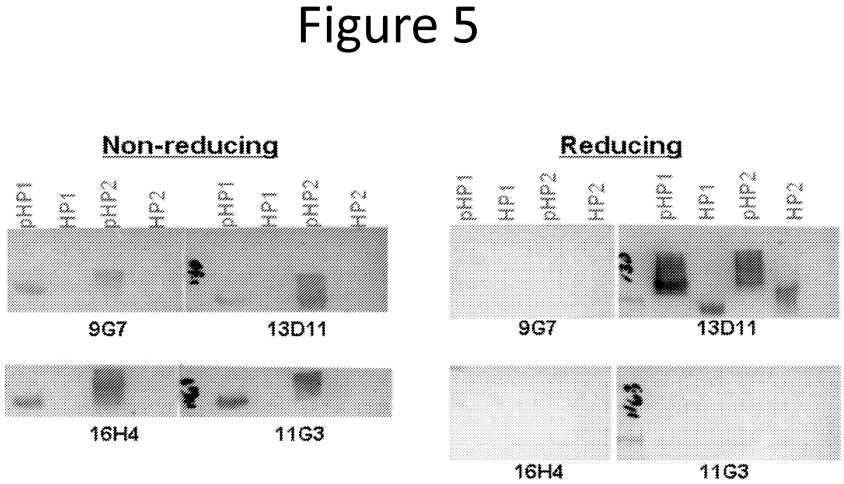

Consistent with the above data, non-denaturing (native) western blots were prepared from polyacrylamide gel electrophoresis (PAGE) and without ("non-reducing") β-mercaptoethanol. Those gels run without SDS and β -mercaptoethanol were considered non-reducing and non-denaturing, leaving the proteins in state believed to retain at least some native three-dimensional and secondary structure. Control gels run without SDS but with samples loaded with β-mercaptoethanol ("reducing") were run and blotted in parallel. As shown in Figure 5, under native (no SDS), non-reducing conditions, all antibodies were capable of recognizing pre-Haptoglobin-1 (preHP1) and pre-Haptoglobin-2 (preHP2), but not mature (processed) haptoglobins. Reduced proteins run under non-denaturing (no SDS) conditions, on the other hand, were only recognized by 13D11.

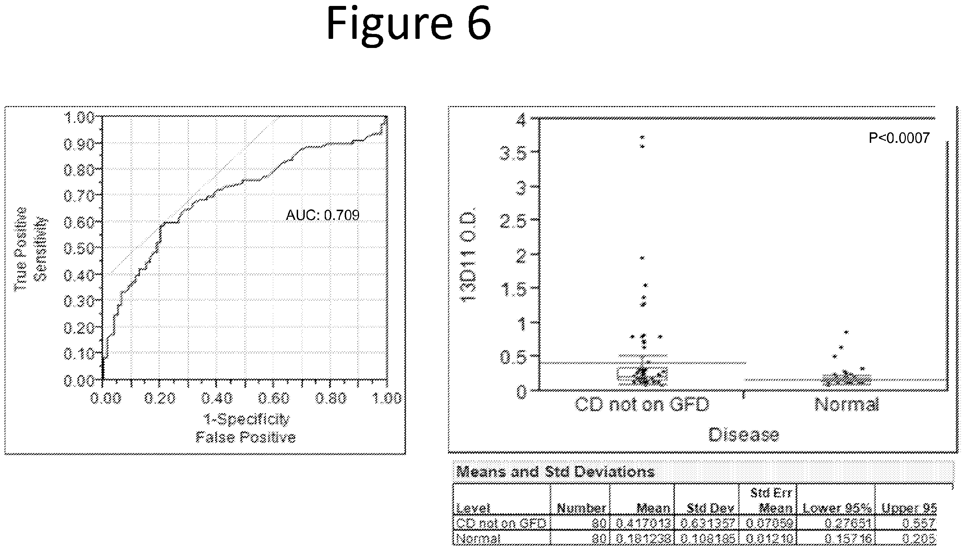

Specificity and sensitivity of antibody pair 13D11+1 1G3-bt were tested. Results are shown in Figure 6. Zonulin levels are increased in patients with celiac disease (Fasano, Ann NY Acad Sci, 2012). That study was done using the anti-Zot antibody in a sandwich ELISA format. Using our new ELISA (13D11+11G3-bt pair), we conducted a similar study using 80 celiac patients and 80 healthy controls. The preHP2 mean OD for celiac disease was significantly higher than that in controls (p<0.0007; t-test). The area under the ROC curve was 0.709. Thus, the new antibody pair allows one to observe elevated preHP2 levels in celiac disease patients.

Results for detection of pre-Haptoglobin-2 by antibody pair 13D11+1 1G3-bt was compared to binding by a commercial polyclonal-antibody-based Zonulin assay (Alpco Zonulin ELISA, Alpco, Salem, New Hampshire) performed according to manufacturer's directions. The Alpco Zonulin assay is a competitive ELISA, which uses a polyclonal anti-zonulin antibody to capture native zonulin in the presence of a biotinylated competitor, presumably recombinant zonulin protein (composition of the competitor is not disclosed by the manufacturer). High signal from the competitor means that native zonulin is not present at substantial amounts in the sample, while low signal in the assay means that high levels of native zonulin is present (inverse correlation of signal to amount of target in the sample being assayed).

Pre-HP2 capture antibody (clone 13D11) was diluted to 2 µg/mL in PBS (10 mM Sodium Phosphate, 150 mM NaCl, pH 7.8) and added to a 96-well plates (100 µl/well; Maxisorp, Nunc, Roskilde, Denmark). After an overnight incubation at 4 °C, antibody solution was removed and wells were blocked with blocking buffer (PBS + 3 % BSA) for one hour at room temperature (RT). Serum samples and calibrators diluted in sample diluent (PBS + 0.1 % Tween-20 + 0.1 %BSA + 0.3 µg/ml mouse IgG (Meridian Life Science, Memphis, TN)) were then added to the plate (100 µl/well) after removal of the blocking buffer and incubated for two hours at RT. The plate was then washed three times with PBS + 0.1 % Tween-20 (PBST) using an automated plate washer (BioPlex Pro II Wash Station, Bio-Rad, Hercules, CA). Next, biotinylated detection antibody (clone 11G3) diluted to 2 ug/ml in conjugate diluent (sample diluent without mouse IgG) was added (100 µl/well) and incubated for one hour at RT. The plate was again washed three times in PBST, and streptavidin-HRP (Thermo Fisher) diluted 1:10,000 in conjugate diluent was added (100 µl/well) and incubated for one hour at RT. After washing, Ultra TMB ELISA substrate (Thermo Fisher) was added to the plate (100 µl/well) and color was allowed to develop for 15 min at RT. Color development was stopped using 10 % v/v sulfuric acid (Ricca Chemical, Arlington, TX) and the optical density (OD) was read at 450 nm using a Benchmark Plus plate reader (Bio-Rad). Data was analyzed using Microplate Manager software (Bio-Rad).

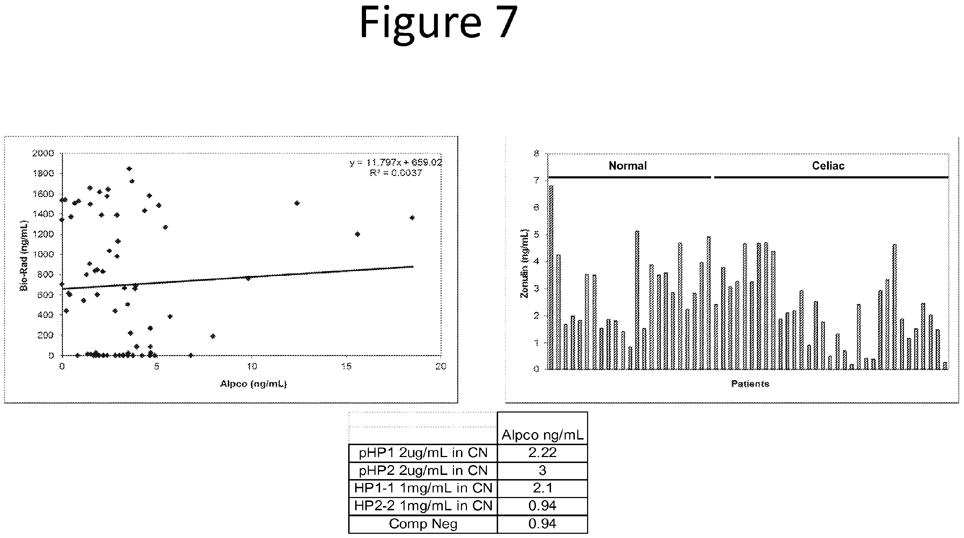

Samples from 23 normal and 47 celiac patients were tested using the Alpco Zonulin ELISA and the antibody pair 13D11+1 1G3-bt. preHP2 was spiked into serum at 2 ug/ml and then this sample was diluted according to the manufacturer's directions. preHP1 (2 µg/ml), HP1-1(1 mg/ml) and HP2-2(1 mg/ml) were also spiked and assayed. No difference was seen between preHP2 signal and the other haptoglobins indicating that the Alpco assay is not specifically detecting preHP2. The table at the bottom of Figure 7 summarizes the results of the assay.

Controls and standards performed as expected in the Alpco assay. However, no correlation was observed between the two assay methods (Figure 7, graph on left). Notably, the Alpco assay failed to detect celiac disease and recombinant protein (Figure 7, graph on right), whereas the antibody pair as described herein accurately detected celiac disease and the recombinant protein.

Seventy six healthy blood donors were tested for pre-HP2 levels using the ELISA. Concentrations for 7 (9 %) of the patients fell below the limit of detection for the assay, and 13 (17 %) samples with high signals required further dilutions to fall within the calibrator curve. The mean pre-HP2 serum level was found to be 221.2 ng/ml (95 % CI: 106.5 - 335.9 ng/ml) with a maximum value of 3165.6 ng/ml. The distribution of pre-HP2 concentrations in the cohort was determined to be non-Gaussian (p<0.01) with a median value of 23.9 ng/ml.

## Claims

1. An isolated monoclonal antibody that binds human pre-Haptoglobin-2 but does not bind human pre-Haptoglobin-1, human Haptoglobin-1, or human Haptoglobin-2 as determined by surface plasmon resonance, wherein the monoclonal antibody specifically binds GYVEHSVRY (SEQ ID NO:1).

2. The isolated monoclonal antibody of claim 1, wherein the monoclonal antibody binds pre-Haptoglobin-2 with a K_{D} of less than 25 or 10 nM.

3. The isolated monoclonal antibody of claim 1, wherein the monoclonal antibody is linked to a detectable label.

4. The isolated monoclonal antibody of claim 3, wherein the detectable label is biotin.

5. The isolated monoclonal antibody of claim 1, wherein the monoclonal antibody is linked to a solid support.

6. A kit comprising the monoclonal antibody of claim 1, wherein the monoclonal antibody is linked to a solid support.

7. The kit of claim 6, further comprising a second antibody that binds human pre-Haptoglobin-2, wherein the monoclonal antibody linked to the solid support and the second antibody can simultaneously bind to human pre-Haptoglobin-2.

8. The kit of claim 7, wherein the second monoclonal antibody is linked to a detectable label.

9. The kit of claim 8, wherein the detectable label is biotin.

10. The kit of claim 7, further comprising avidin linked to a second detectable label.

11. The kit of claim 7, further comprising a detectably-labeled secondary antibody that binds the second monoclonal antibody.

12. A method of detecting human pre-Haptoglobin-2 in a sample, the method comprising,
contacting the sample with a monoclonal antibody of claim 1 under conditions such that the antibody binds to human pre-Haptoglobin-2, if present in the sample; and detecting the presence, absence, or quantity of binding of the antibody to human pre-Haptoglobin-2 from the sample.

13. The method of claim 12, wherein the monoclonal antibody is linked to a solid support.

14. The method of claim 13, wherein after the contacting, unbound components of the sample are washed away from the antibody linked to the solid support while human pre-Haptoglobin-2, if present, remains bound to the antibody, and the method further comprises contacting the human pre-Haptoglobin-2 bound to the antibody linked to the solid support with a second monoclonal antibody that binds human pre-Haptoglobin-2, and wherein the detecting comprises detecting the presence, absence, or quantity of the second monoclonal antibody.

15. The method of claim 12, wherein the antibody is detectably-labeled and the detecting comprises detecting the detectably-labeled antibody with a microscope.

## Patentansprüche

1. Isolierter monoklonaler Antikörper, der humanes prä-Haptoglobin-2 bindet, aber kein humanes prä-Haptoglobin-1, humanes Haptoglobin-1 oder humanes Haptoglobin-2 bindet, wie es durch Oberflächenplasmonenresonanz bestimmt wird, wobei der monoklonale Antikörper spezifisch GYVEHSVRY (SEQ ID Nr. 1) bindet.

2. Isolierter monoklonaler Antikörper gemäß Anspruch 1, wobei der monoklonale Antikörper prä-Haptoglobin-2 mit einem K_{D} von weniger als 25 oder 10 nM bindet.

3. Isolierter monoklonaler Antikörper gemäß Anspruch 1, wobei der monoklonale Antikörper mit einem nachweisbaren Marker verknüpft ist.

4. Isolierter monoklonaler Antikörper gemäß Anspruch 3, wobei es sich bei dem nachweisbaren Marker um Biotin handelt.

5. Isolierter monoklonaler Antikörper gemäß Anspruch 1, wobei der monoklonale Antikörper mit einem festen Träger verknüpft ist.

6. Kit, der den monoklonalen Antikörper gemäß Anspruch 1 umfasst, wobei der monoklonale Antikörper mit einem festen Träger verknüpft ist.

7. Kit gemäß Anspruch 6, weiterhin umfassend einen zweiten Antikörper, der humanes prä-Haptoglobin-2 bindet, wobei der mit dem festen Träger verknüpfte monoklonale Antikörper und der zweite Antikörper gleichzeitig an humanes prä-Haptoglobin-2 binden können.

8. Kit gemäß Anspruch 7, wobei der zweite monoklonale Antikörper mit einem nachweisbaren Marker verknüpft ist.

9. Kit gemäß Anspruch 8, wobei es sich bei dem nachweisbaren Marker um Biotin handelt.

10. Kit gemäß Anspruch 7, weiterhin umfassend Avidin, das mit einem zweiten nachweisbaren Marker verknüpft ist.

11. Kit gemäß Anspruch 7, weiterhin umfassend einen nachweisbar markierten sekundären Antikörper, der den zweiten monoklonalen Antikörper bindet.

12. Verfahren zum Nachweisen von humanem prä-Haptoglobin-2 in einer Probe, wobei das Verfahren umfasst:
In-Kontakt-Bringen der Probe mit einem monoklonalen Antikörper gemäß Anspruch 1 unter Bedingungen, bei denen der Antikörper an humanes prä-Haptoglobin-2 bindet, wenn welches in der Probe vorhanden ist; und
Nachweisen der Anwesenheit, der Abwesenheit oder der Menge der Bindung des Antikörpers an humanes prä-Haptoglobin-2 aus der Probe.

13. Verfahren gemäß Anspruch 12, wobei der monoklonale Antikörper mit einem festen Träger verknüpft ist.

14. Verfahren gemäß Anspruch 13, wobei nach dem In-Kontakt-Bringen ungebundene Komponenten der Probe von dem mit dem festen Träger verknüpften Antikörper weg gewaschen werden, während humanes prä-Haptoglobin-2, falls vorhanden, an den Antikörper gebunden bleibt, und das Verfahren weiterhin das In-Kontakt-Bringen des humanen prä-Haptoglobin-2, das an den mit dem festen Träger verknüpften Antikörper gebunden ist, mit einem zweiten monoklonalen Antikörper, der humanes prä-Haptoglobin-2 bindet, umfasst und wobei das Nachweisen das Nachweisen der Anwesenheit, der Abwesenheit oder der Menge des zweiten monoklonalen Antikörpers umfasst.

15. Verfahren gemäß Anspruch 12, wobei der Antikörper nachweisbar markiert ist und das Nachweisen das Nachweisen des nachweisbar markierten Antikörpers mit einem Mikroskop umfasst.

## Revendications

1. Anticorps monoclonal isolé qui se lie à la pré-Haptoglobine-2 humaine mais ne se lie ni à la pré-Haptoglobine-1 humaine, ni à l'Haptoglobine-l humaine ni à l'Haptoglobine-2 humaine, tel que déterminé par résonance plasmonique de surface, ledit anticorps monoclonal se liant spécifiquement à GYVEHSVRY (SEQ ID NO : 1).

2. Anticorps monoclonal isolé selon la revendication 1, ledit anticorps monoclonal se liant à la pré-Haptoglobine-2 avec un K_{D} inférieur à 25 ou 10 nM.

3. Anticorps monoclonal isolé selon la revendication 1, ledit anticorps monoclonal étant lié à un marqueur détectable.

4. Anticorps monoclonal isolé selon la revendication 3, où le marqueur détectable est la biotine.

5. Anticorps monoclonal isolé selon la revendication 1, ledit anticorps monoclonal étant lié à un support solide.

6. Trousse contenant l'anticorps monoclonal selon la revendication 1, dans laquelle l'anticorps monoclonal est lié à un support solide.

7. Trousse selon la revendication 6, contenant en outre un deuxième anticorps qui se lie à la pré-Haptoglobine-2 humaine, dans laquelle l'anticorps monoclonal lié au support solide et le deuxième anticorps peuvent se lier simultanément à la pré-Haptoglobine-2 humaine.

8. Trousse selon la revendication 7, dans laquelle le deuxième anticorps monoclonal est lié à un marqueur détectable.

9. Trousse selon la revendication 8, dans laquelle le marqueur détectable est la biotine.

10. Trousse selon la revendication 7, contenant en outre de l'avidine liée à un deuxième marqueur détectable.

11. Trousse selon la revendication 7, contenant en outre un anticorps secondaire marqué de façon détectable, qui se lie au deuxième anticorps monoclonal.

12. Procédé de détection de la pré-Haptoglobine-2 humaine dans un échantillon, ledit procédé comprenant :
la mise en contact de l'échantillon avec un anticorps monoclonal selon la revendication 1 dans des conditions telles que l'anticorps se lie à la pré-Haptoglobine-2 humaine si elle est présente dans l'échantillon ; et
la détection de la présence, de l'absence ou de la quantité de liaison de l'anticorps à la pré-Haptoglobine-2 humaine de l'échantillon.

13. Procédé selon la revendication 12, dans lequel l'anticorps monoclonal est lié à un support solide.

14. Procédé selon la revendication 13, dans lequel après la mise en contact, les composants non liés de l'échantillon sont éliminés de l'anticorps lié au support solide par lavage tandis que, si elle est présente, la pré-Haptoglobine-2 humaine reste liée à l'anticorps, et le procédé comprend en outre la mise en contact de la pré-Haptoglobine-2 humaine liée à l'anticorps, qui est lui-même lié au support solide, avec un deuxième anticorps monoclonal qui se lie à la pré-Haptoglobine-2 humaine, et dans lequel la détection comprend la détection de la présence, de l'absence ou de la quantité du deuxième anticorps monoclonal.

15. Procédé selon la revendication 12, dans lequel l'anticorps est marqué de façon détectable et la détection comprend la détection au microscope de l'anticorps marqué de façon détectable.


### SEQUENCE LISTING

<110> Bio-Rad Laboratories, Inc.
<120> Pre-Haptoglobin-2 Monoclonal Antibodies
   and Uses Thereof
<130> 95191-923192
<140> WO Not yet assigned
   <141> Not yet assigned
<150> US 61/900,001
   <151> 2013-11-05
<160> 15
<170> FastSEQ for Windows Version 4.0
<210> 1
   <211> 9
   <212> PRT
   <213> Artificial Sequence
<220>
   <223> synthetic anti-human pre-haptoglobin-2 monoclonal antibody 13D11 minimal epitope
<400> 1
   Gly Tyr Val Glu His Ser Val Arg Tyr
    1               5
<210> 2
   <211> 23
   <212> PRT
   <213> Artificial Sequence
<220>
   <223> synthetic human pre-haptoglobin-2 peptide bound by anti-human pre-haptoglobin-2 monoclonal antibody 13D11
<400> 2
   Lys Pro Pro Glu Ile Ala His Gly Tyr Val Glu His Ser Val Arg Tyr
    1               5                  10                  15
   Gln Cys Lys Asn Tyr Tyr Lys
               20
<210> 3
   <211> 15
   <212> PRT
   <213> Artificial Sequence
<220>
   <223> synthetic human pre-haptoglobin-2 peptide 35,94 bound by anti-human pre-haptoglobin-2 monoclonal antibody 13D11
<400> 3
   Lys Pro Pro Glu Ile Ala His Gly Tyr Val Glu His Ser Val Arg
    1               5                  10                  15
<210> 4
   <211> 15
   <212> PRT
   <213> Artificial Sequence
<220>
   <223> synthetic human pre-haptoglobin-2 peptide 36,95 bound by anti-human pre-haptoglobin-2 monoclonal antibody 13D11
<400> 4
   Pro Pro Glu Ile Ala His Gly Tyr Val Glu His Ser Val Arg Tyr
    1               5                  10                  15
<210> 5
   <211> 15
   <212> PRT
   <213> Artificial Sequence
<220>
   <223> synthetic human pre-haptoglobin-2 peptide 37,96 bound by anti-human pre-haptoglobin-2 monoclonal antibody 13D11
<400> 5
   Pro Glu Ile Ala His Gly Tyr Val Glu His Ser Val Arg Tyr Gln
    1               5                  10                  15
<210> 6
   <211> 15
   <212> PRT
   <213> Artificial Sequence
<220>
   <223> synthetic human pre-haptoglobin-2 peptide 38,97 bound by anti-human pre-haptoglobin-2 monoclonal antibody 13D11
<400> 6
   Glu Ile Ala His Gly Tyr Val Glu His Ser Val Arg Tyr Gln Cys
   1               5                   10                  15
<210> 7
   <211> 15
   <212> PRT
   <213> Artificial Sequence
<220>
   <223> synthetic human pre-haptoglobin-2 peptide 39,98 bound by anti-human pre-haptoglobin-2 monoclonal antibody 13D11
<400> 7
   Ile Ala His Gly Tyr Val Glu His Ser Val Arg Tyr Gln Cys Lys
   1               5                   10                  15
<210> 8
   <211> 15
   <212> **PRT**
   <213> **Artificial** Sequence
<220>
   <223> synthetic human pre-haptoglobin-2 peptide 40,99 bound by anti-human pre-haptoglobin-2 monoclonal antibody 13D11
<400> 8
   Ala His Gly Tyr Val Glu His Ser Val Arg Tyr Gln Cys Lys Asn
   1               5                   10                  15
<210> 9
   <211> 15
   <212> PRT
   <213> Artificial Sequence
<220>
   <223> synthetic human pre-haptoglobin-2 peptide 41,100 bound by anti-human pre-haptoglobin-2 monoclonal antibody 13D11
<400> 9
   His Gly Tyr Val Glu His Ser Val Arg Tyr Gln Cys Lys Asn Tyr
   1               5                   10                  15
<210> 10
   <211> 15
   <212> **PRT**
   <213> Artificial Sequence
<220>
   <223> synthetic human pre-haptoglobin-2 peptide 42,101 bound by anti-human pre-haptoglobin-2 monoclonal antibody 13D11
<400> 10
   Gly Tyr Val Glu His Ser Val Arg Tyr Gln Cys Lys Asn Tyr Tyr
    1               5                  10                  15
<210> 11
   <211> 15
   <212> PRT
   <213> Artificial Sequence
<220>
   <223> synthetic human pre-haptoglobin-2 peptide 43,102 bound by anti-human pre-haptoglobin-2 monoclonal antibody 13D11
<400> 11
   Tyr Val Glu His Ser Val Arg Tyr Gln Cys Lys Asn Tyr Tyr Lys
    1               5                  10                  15
<210> 12
   <211> 15
   <212> PRT
   <213> Artificial Sequence
<220>
   <223> synthetic human pre-haptoglobin-2 peptide 34,93 not bound by anti-human pre-haptoglobin-2 monoclonal antibody 13D11
<400> 12
   Pro Lys Pro Pro Glu Ile Ala His Gly Tyr Val Glu His Ser Val
    1               5                  10                  15
<210> 13
   <211> 15
   <212> PRT
   <213> Artificial Sequence
<220>
   <223> synthetic human pre-haptoglobin-2 peptide 44,103 not bound by anti-human pre-haptoglobin-2 monoclonal antibody 13D11
<400> 13
   Val Glu His Ser Val Arg Tyr Gln Cys Lys Asn Tyr Tyr Lys Leu
    1               5                  10                  15
<210> 14
   <211> 6
   <212> PRT
   <213> Artificial Sequence
<220>
   <223> synthetic C-terminal hexahistidine tag, His-tag
<400> 14
   His His His His His His
    1                   5
<210> 15
   <211> 406
   <212> PRT
   <213> Homo sapiens
<220>
   <223> human pre-haptoglobin-2 (preHP2), haptoglobin preproprotein, isoform 1 (HP, HP2ALPHA2), haptoglobin alpha(1S)-beta (HPA1S), haptoglobin alpha(2FS)-beta, zonulin, binding peptide (BP)
<220>
   <221> SIGNAL
   <222> (1)...(18)
   <223> signal sequence, signal peptide
<220>
   <221> CDS
   <222> (1)..(406)
   <223> human pre-haptoglobin-2 (preHP2)
<220>
   <221> PROPEP
   <222> (19)...(406)
   <223> haptoglobin proprotein
<220>
   <221> CHAIN
   <222> (19)...(160)
   <223> haptoglobin alpha-chain mature peptide
<220>
   <221> REPEAT
   <222> (29)...(46)
   <223> haptoglobin alpha-chain repeat sequence
<220>
   <221> CHAIN
   <222> (162)...(406)
   <223> haptoglobin beta-chain mature peptide
<400> 15
   Met Ser Ala Leu Gly Ala Val Ile Ala Leu Leu Leu Trp Gly Gln Leu
    1               5                  10                  15
   Phe Ala Val Asp Ser Gly Asn Asp Val Thr Asp Ile Ala Asp Asp Gly
               20                  25                  30
   Cys Pro Lys Pro Pro Glu Ile Ala His Gly Tyr Val Glu His Ser Val
           35                  40                  45
   Arg Tyr Gln Cys Lys Asn Tyr Tyr Lys Leu Arg Thr Glu Gly Asp Gly
       50                  55                  60
   Val Tyr Thr Leu Asn Asp Lys Lys Gln Trp Ile Asn Lys Ala Val Gly
   65                  70                  75                  80
   Asp Lys Leu Pro Glu Cys Glu Ala Asp Asp Gly Cys Pro Lys Pro Pro
                   85                  90                  95
   Glu Ile Ala His Gly Tyr Val Glu His Ser Val Arg Tyr Gln Cys Lys
               100                 105                 110
   Asn Tyr Tyr Lys Leu Arg Thr Glu Gly Asp Gly Val Tyr Thr Leu Asn
           115                 120                 125
   Asn Glu Lys Gln Trp Ile Asn Lys Ala Val Gly Asp Lys Leu Pro Glu
       130                 135                 140
   Cys Glu Ala Val Cys Gly Lys Pro Lys Asn Pro Ala Asn Pro Val Gln
   145                 150                 155                 160
   Arg Ile Leu Gly Gly His Leu Asp Ala Lys Gly Ser Phe Pro Trp Gln
                   165                 170                 175
   Ala Lys Met Val Ser His His Asn Leu Thr Thr Gly Ala Thr Leu Ile
               180                 185                 190
   Asn Glu Gln Trp Leu Leu Thr Thr Ala Lys Asn Leu Phe Leu Asn His
           195                 200                 205
   Ser Glu Asn Ala Thr Ala Lys Asp Ile Ala Pro Thr Leu Thr Leu Tyr
       210                 215                 220
   Val Gly Lys Lys Gln Leu Val Glu Ile Glu Lys Val Val Leu His Pro
   225                 230                 235                 240
   Asn Tyr Ser Gln Val Asp Ile Gly Leu Ile Lys Leu Lys Gln Lys Val
                   245                 250                 255
   Ser Val Asn Glu Arg Val Met Pro Ile Cys Leu Pro Ser Lys Asp Tyr
               260                 265                 270
   Ala Glu Val Gly Arg Val Gly Tyr Val Ser Gly Trp Gly Arg Asn Ala
           275                 280                 285
   Asn Phe Lys Phe Thr Asp His Leu Lys Tyr Val Met Leu Pro Val Ala
       290                 295                 300
   Asp Gln Asp Gln Cys Ile Arg His Tyr Glu Gly Ser Thr Val Pro Glu
   305                 310                 315                 320
   Lys Lys Thr Pro Lys Ser Pro Val Gly Val Gln Pro Ile Leu Asn Glu
                   325                 330                 335
   His Thr Phe Cys Ala Gly Met Ser Lys Tyr Gln Glu Asp Thr Cys Tyr
               340                 345                 350
   Gly Asp Ala Gly Ser Ala Phe Ala Val His Asp Leu Glu Glu Asp Thr
           355                 360                 365
   Trp Tyr Ala Thr Gly Ile Leu Ser Phe Asp Lys Ser Cys Ala Val Ala
       370                 375                 380
   Glu Tyr Gly Val Tyr Val Lys Val Thr Ser Ile Gln Asp Trp Val Gln
   385                 390                 395                 400
   Lys Thr Ile Ala Glu Asn
                   405